# EUROPEAN PATENT SPECIFICATION

(11) **EP 3 412 765 B1**
(45) Date of publication and mention of the grant of the patent: **18.01.2023**
(21) Application number: 17747472.3
(22) Date of filing: 01.02.2017
(51) Int. Cl.: C12N 15/10, C12N 9/22, C12N 1/15, C12P 7/40, C12N 15/90

(54) **METHOD FOR PRODUCING MUTANT FILAMENTOUS FUNGI**
VERFAHREN ZUR HERSTELLUNG VON MUTANTEN FADENPILZEN
PROCÉDÉ DE PRODUCTION DE CHAMPIGNONS FILAMENTEUX MUTANTS

(30) Priority: 04.02.2016 JP 2016019676; 03.08.2016 JP 2016152972
(43) Date of publication of application: 12.12.2018
(73) Proprietor: KAO CORPORATION, Chuo-ku Tokyo 103-8210 (JP)
(72) Inventor: TSUBOI, Yuichi, Wakayama-shi Wakayama 640-8580 (JP)
(74) Representative: Vossius & Partner Patentanwälte Rechtsanwälte mbB
(86) International application number: PCT/JP2017/003648
(87) International publication number: WO 2017/135317

(56) References cited:
- WO-A1-2015/017866
- WO-A1-2016/110453
- JP-A- 2002 065 273
- JP-A- 2015 192 662
- JP-A- 2015 519 923
- TORU MATSU-URA ET AL: "Efficient gene editing inNeurospora crassa withCRISPR technology", FUNGAL BIOL BIOTECHNOL, vol. 2, 1 January 2015 (2015-01-01), page 4, XP055268999, DOI: 10.1186/s40694-015-0015-1
- KATAYAMA TAKUYA ET AL: "Development of a genome editing technique using the CRISPR/Cas9 system in the industrial filamentous fungusAspergillus oryzae", BIOTECHNOLOGY LETTERS, KLUWER ACADEMIC PUBLISHERS, DORDRECHT, vol. 38, no. 4, 19 December 2015 (2015-12-19), pages 637-642, XP035901398, ISSN: 0141-5492, DOI: 10.1007/S10529-015-2015-X [retrieved on 2015-12-19]
- DEWEI JIANG ET AL: "Molecular tools for functional genomics in filamentous fungi: Recent advances and new strategies", BIOTECHNOLOGY ADVANCES., vol. 31, no. 8, 1 December 2013 (2013-12-01), pages 1562-1574, XP055250956, GB ISSN: 0734-9750, DOI: 10.1016/j.biotechadv.2013.08.005
- CHRISTINA S. NØDVIG ET AL: "A CRISPR-Cas9 System for Genetic Engineering of Filamentous Fungi", PLOS ONE, vol. 10, no. 7, 15 July 2015 (2015-07-15), pages 1-18, XP055256394, DOI: 10.1371/journal.pone.0133085
- LIU, R. et al.: "Efficient genome editing in filamentous fungus Trichoderma reesei using the CRISPR/Cas9 system", Cell Discovery, vol. 1, no. 15007, 2015, pages 1-11, XP002755316, ISSN: 2056-5968, DOI: doi:10.1038/celldisc.2015.7
- ARAZOE, T. et al.: "Tailor-made Talen system for highly efficient targeted gene replacement in the rice blast fungus", Biotechnology and Bioengineering, vol. 112, no. 7, 2015, pages 1335-1342, XP055579073, ISSN: 0006-3592, DOI: doi:10.1002/bit.25559
- ARAZOE, T. et al.: "Tailor-made CRISPR/Cas system for highly efficient targeted gene replacement in the rice blast fungus", Biotechnology and Bioengineering, vol. 112, no. 12, 2015, pages 2543-2549, XP055256237, ISSN: 0006-3592, DOI: doi:10.1002/bit.25662
- CHEN, F. et al.: "High frequency genome editing using ssDNA oligonucleotides with zinc-finger nucleases", Nature Methods, vol. 8, no. 9, 2011, pages 753-755, XP002681301, ISSN: 1548-7091, DOI: doi:10.1038/NMETH.1653
- FAGERLUND, R.D. et al.: "The Cpfl CRISPR-Cas protein expands genome-editing tools", Genome Biology, vol. 16, no. 251, 2015, pages 1-3, XP002757560, ISSN: 1474-7596
- SKORY, C.D. et al.: "Homologous recombination and double-strand break repair in the transformation of Rhizopus oryzae", Mol. Genet. Genomics, vol. 268, 2002, pages 397-406, XP002282394, ISSN: 1617-4615, DOI: doi:10.1007/s00438-002-0760-8
- Tsuboi, Yuichi et al.: "Jinko DNA Setsudan Koso TALEN o Mochiita Rhizopus oryzae ni Okeru Idenshi Hakai Gijutsu no Kakuritsu", 2016 Nendo Proceedings of the Annual Meeting of Japan Society for Cioscience, Biotechnology, and Agrochemistry, no. 3F216, 5 March 2016 (2016-03-05), ISSN: 2186-7976
- NAKADE, S. et al.: "Microhomology-mediated end-joining-dependent integration of donor DNA in cells and animals using TALENs and CRISPR/Cas9", Nature Communications, vol. 5, no. 5560, 2014, pages 1-8, ISSN: 2041-1723

## Description

### [Background]

In recent years, genome editing technology using a programmable nuclease has been reported. The genome editing is a technique of engineering a genome in a site-directed manner by specifically cleaving the DNA duplex of a target gene on the genome and inducing deletion, insertion or substitution of a nucleotide(s) in the course of repair of the cleaved DNA or inserting a foreign polynucleotide(s) thereto, for example. Such a technique is known as TALEN (transcription activator-like effector nuclease), ZFN (zinc-finger nuclease), CRISPR (clustered regularly interspaced short palindromic repeat)-Cas9, CRISPR-Cpf1, homing endonuclease, compact designer TALEN, etc. It has been reported that the genome editing using a programmable nuclease can improve homologous recombination efficiency of a target site on genomic DNA and is capable of causing homologous recombination even in organisms originally having the low ability of homologous recombination (Non Patent Literature 1).

The genome editing technology has improved homologous recombination efficiency of genes of mammalian cells which have heretofore been relatively difficult to be engineered. Non Patent Literature 2 reports an approach of researching single-nucleotide polymorphism by engineering one base of a target genomic region of human using single-stranded DNA transferred to the genome with the TALEN technology. Non Patent Literature 3 reports an approach of engineering a gene by transferring single-stranded DNA to the genome of mouse embryonic cells by use of the TALEN technology. However, in this method, reportedly, a genomic region having from one to several bases, up to shorter than 300 bp was merely substituted or deleted. Non Patent Literature 4 reports an approach of engineering a gene by transferring single-stranded DNA to the genome of rat embryonic cells by use of the CRISPR-Cas9 technology and describes that DNA of approximately 830 bp at the maximum was inserted, and that two single-stranded DNAs are necessary for insertion or substitution of a genomic region having a longer chain. However, in this method as well, it is still difficult to insert one gene region itself to the genome or replace an existing large genomic region with a different gene region.

Filamentous fungi such as *Rhizopus* fungi are useful microorganisms which can be used in microbiological production of useful substances such as organic acids. There is a demand for development of mutants of filamentous fungi more suitable for production of useful substance by gene recombination techniques (Patent Literature 1).

A filamentous fungus *Neurospora crassa* is an organism which is often used in recombination research. However, wild-type *Neurospora crassa* has a very low rate of homologous recombination and is thus not easy to be genetically engineered by a homologous recombination method.

Homologous recombination in the fungi of the genus *Rhizopus* is not easy either. It is a rare occurrence in the fungi of the genus *Rhizopus* to introduce a DNA fragment transferred to cells into genomic DNA through homologous recombination. Also, in the fungi of the genus *Rhizopus,* the transferred DNA fragment is spontaneously cyclized or amplified without a replication origin. Therefore, it is difficult to screen for recombinant strains using a gene marker. Furthermore, genetic backgrounds of fungi of the genus *Rhizopus* still remain to be fully studied. Thus, conventional development of mutants of the genus *Rhizopus* has been associated with technical difficulty. Methods based on targeting to homologous sequence portions, such as an Agrobacterium method (Non Patent Literature 5) and a method of terminally modifying DNA to be transferred to cells (Non Patent Literature 6) have been attempted so far for gene transfer to the genome of fungi of the genus *Rhizopus.* However, these methods are not efficient. Meanwhile, there is only one report on a gene-disrupted fungal strain of the genus *Rhizopus* (Non Patent Literature 7).

(Non-Patent Literature 1) Science, 2013, 339 (6121): 823-826
(Non-Patent Literature 2) Int J Mol Sci, 2015, 16: 21128-21137
(Non-Patent Literature 3) PNAS, 2013, 110 (10): 3782-87
(Non-Patent Literature 4) Nature Commun, 2016, 20, doi: 10.1038/ncomms10431
(Non-Patent Literature 5) Mol Genet Genomics, 2004, 271 (4): 499-510
(Non-Patent Literature 6) Mol Genet Genomics, 2005, 274 (4): 373-383
(Non-Patent Literature 7) Mol Microbiol, 2010, 77 (3): 587-604
(Patent Literature 1)WO2016/110453

### [Summary]

Described is a method for producing a mutant filamentous fungus, comprising transferring a programmable DNA nuclease and single-stranded DNA to a host filamentous fungus, and substituting an upstream region and a downstream region of a cleavage site for the programmable DNA nuclease in genomic DNA of the host by the single-stranded DNA through homologous recombination. The invention relates to the embodiments as defined in the claims. In particular, the invention relates to a method for producing a mutant filamentous fungus, comprising transferring a programmable DNA nuclease and single-stranded DNA into a host filamentous fungus, and substituting an upstream region and a downstream region of a cleavage site for the programmable DNA nuclease in genomic DNA of the host by the single-stranded DNA through homologous recombination, wherein the filamentous fungus is a fungus of the genus *Rhizopus.*

### [Detailed Description]

In the present specification, the terms "upstream" and "downstream" as to a gene or DNA refer to upstream and downstream in the direction of transcription of the gene or the DNA unless otherwise specified.

In the present specification, the term "operable linking" between a gene and a control region refers to the linking between the gene and the control region such that the gene can be expressed under control of the control region. The procedures of the "operable linking" between a gene and a control region are well known to those skilled in the art.

The term "genome editing" is a technique of engineering a genome in a target site-directed manner by specifically cleaving the DNA duplex of a target gene locus on the genome using a programmable artificial DNA nuclease and inducing deletion, insertion or substitution of nucleotides in the course of repair of the cleaved DNA or inserting a foreign polynucleotide thereto, for example. Such a genome editing technique is known as TALEN (transcription activator-like effector nuclease), ZFN (zinc-finger nuclease), CRISPR (clustered regularly interspaced short palindromic repeat)-Cas9, CRISPR-Cpf1, homing endonuclease, compact designer TALEN, etc. typically named after the programmable artificial DNA nuclease used (Nature Reviews Genetics, 2014, 15: 321-334; Nucleic Acids Research, 2011, 39: e82; Nucleic Acids Research, 2006, 34: e149; and Nature communications, 2013, 4: 1762). A kit for the genome editing based on these techniques is commercially available and can be purchased from, for example, Life Technologies Corp., Cellectis, or Transposagen Biopharmaceuticals, Inc.

The present inventor succeeded in preparing mutant filamentous fungi more efficiently than ever by applying the genome editing technology using a programmable DNA nuclease to filamentous fungi. However, the rate of recombination was still insufficient. In particular, the engineering of a large region of the genome, such as deletion or substitution of the whole of one gene region, was difficult.

The present inventor conducted further studies on homologous recombination techniques based on the genome editing of filamentous fungi and consequently found that use of single-stranded DNA as donor DNA to be transferred to cells together with a programmable DNA nuclease improves the rate of homologous recombination and further allows a long-chain region of the genome to be engineered.

Thus, the present specification describes a method for producing a mutant filamentous fungus. The method is based on the genome editing technology and comprises transferring a programmable DNA nuclease and single-stranded DNA to a host filamentous fungus, and substituting an upstream region and a downstream region of a cleavage site for the programmable DNA nuclease in genomic DNA of the host by the single-stranded DNA through homologous recombination.

Thus, a mutant filamentous fungus with a long-chain region of the genome engineered can be efficiently produced. The mutant filamentous fungus may be a recombinant filamentous fungus obtained by transferring a heterologous gene to a host, may be a mutant filamentous fungus obtained by transferring a endogenous gene to a host, or may be a mutant filamentous fungus having a deletion mutation in a gene of a host.

Examples of the host filamentous fungus include fungi belonging to the phylum *Zygomycota,* the phylum *Ascomycota,* the phylum *Basidiomycota,* and the phylum *Chytridiomycota.* Examples of the fungus belonging to the phylum *Zygomycota* include fungi of the genus *Rhizopus,* the genus *Mucor,* and the genus *Mortierella.* Examples of the fungus belonging to the phylum *Ascomycota* include fungi of the genus *Acremonium,* the genus *Aspergillus,* the genus *Aureobasidium,* the genus *Chrysosporium,* the genus *Fusarium,* the genus *Magnaporthe,* the genus *Myceliophthora,* the genus *Neurospora,* the genus *Paecilomyces,* the genus *Penicillium,* the genus *Talaromyces,* the genus *Thermoascus,* the genus *Thielavia,* the genus *Tolypocladium,* and the genus *Trichoderma.* Examples of the fungus belonging to the phylum *Basidiomycota* include fungi of the genus *Bjerkandera,* the genus *Ceriporiopsis,* the genus *Coprinus,* the genus *Coriolus,* the genus *Cryptococcus,* the genus *Folibasidium,* the genus *Humicola,* the genus *Phanerochaete,* the genus *Phlebia,* the genus *Pleurotus,* the genus *Schizophyllum,* and the genus *Trametes.* Examples of the fungus belonging to the phylum *Chytridiomycota* include fungi of the genus *Chytridium,* the genus *Nowakovskiella,* the genus *Olpidium,* the genus *Allomyces,* the genus *Coelomomyces,* and the genus *Monoblepharis.* Among them, a fungus belonging to the phylum *Zygomycota* or the phylum *Ascomycota* is preferred, a fungus belonging to the phylum *Zygomycota* is more preferred, and a fungus of the genus *Rhizopus* is still more preferred. Examples of the fungus of the genus *Rhizopus* include *Rhizopus oryzae, Rhizopus arrhizus, Rhizopus chinensis, Rhizopus nigricans, Rhizopus tonkinensis, Rhizopus tritici,* and *Rhizopus delemar.* Among them, *Rhizopus delemar* and *Rhizopus oryzae* are preferred, and *Rhizopus delemar* is more preferred.

The programmable DNA nuclease used in the described methods is a DNA nuclease which specifically recognizes and cleaves a genomic DNA site to be cleaved. Preferably, the programmable DNA nuclease used in the described methods is a programmable artificial DNA nuclease for use in the genome editing technology mentioned above, more preferably a programmable artificial DNA nuclease for use in the TALEN, CRISPR-Cas9, CRISPR-Cpf1, or ZFN technology. Examples thereof include TALEN (transcription activator-like effector nuclease), Cas9 nuclease or Cas9 (D10A) nuclease, Cpf1 nuclease, and ZFN (zinc-finger nuclease). TALEN or ZFN is a complex of a target recognition region specifically binding near the genomic DNA site to be cleaved, and a DNA nuclease, which permits specific recognition and cleavage of the genomic DNA site to be cleaved. On the other hand, in the CRISPR-Cas9 technology, DNA cleavage is induced by using a DNA nuclease Cas9 and a guide RNA which mimics the hairpin structure of tracrRNA-crRNA. In the CRISPR-Cpf1 technology, DNA cleavage is induced by using a DNA nuclease Cpf1 and a guide RNA which mimics crRNA. In this respect, the DNA cleavage specificity of Cas9 or Cpf1 is defined by the guide RNA and permits cleavage at a target site. Those skilled in the art can obtain the DNA sequences of the genomic DNA site to be cleaved and its surroundings and design an artificial DNA nuclease or guide RNA having a target recognition region appropriate therefor.

In the case of transferring a programmable DNA nuclease to a host, its nuclease polyribonucleotide or the nuclease polypeptide may be transferred directly to the host cells. It is preferred to transfer a polynucleotide encoding the nuclease to the host cells and express the nuclease in the cells.

In the case of transferring a polynucleotide encoding the programmable DNA nuclease to a host cells, preferably, an expression vector containing the polynucleotide encoding the DNA nuclease is transferred to the host cells. The expression vector is not particularly limited as long as the expression vector is stably retained in the host cells and is capable of proliferating therein. The expression vector can be appropriately selected by those skilled in the art. Examples of the expression vector suitable for the fungus of the genus *Rhizopus* include pUC18/19, pUC118/119, pBR322, pMW218/219, pPTR1/2 (Takara Bio Inc.), pRI909/910 (Takara Bio Inc.), pDJB2 (D.J. Ballance et al., Gene, 36, 321-331, 1985), pAB4-1 (van Hartingsveldt W et al., Mol Gen Genet, 206, 71-75, 1987), pLeu4 (M.I.G. Roncero et al., Gene, 84, 335-343, 1989), pPyr225 (C.D. Skory et al., Mol Genet Genomics, 268, 397-406, 2002), and pFG1 (Gruber, F. et al., Curr Genet, 18, 447-451, 1990).

For efficient expression of a programmable DNA nuclease in host cells, it is preferred that a polynucleotide encoding the programmable DNA nuclease should be operably linked to a promoter which functions in the host cells. The type of the promoter used can be appropriately selected by those skilled in the art according to the host cells. Examples of the promoter which functions in the fungus of the genus *Rhizopus* include ldhA promoter (U.S. Patent No. 6268189), pgk1 promoter (WO 2001/73083), pgk2 promoter (WO 2001/72967), pdcA promoter and amyA promoter (Archives of Microbiology, 2006, 186: 41-50), tef and 18S rRNA promoters (U.S. Patent Application Publication No. 2010/112651), and adh1 promoter (JP-A-2015-155759).

In the case of using the programmable DNA nuclease, disruption, etc. of target DNA by the programmable DNA nuclease is promoted by expressing a foreign exonuclease in a host (Scientific Reports, 2013, 3: 1253, DOI: 10.1038/srep01253; and Nat Methods, 2012, 9: 973-975). Thus, preferably, an exonuclease or a polynucleotide encoding the exonuclease is transferred to host cells together with the programmable DNA nuclease or the polynucleotide encoding the programmable DNA nuclease, followed by co-expression thereof in the host cells. The exonuclease is not particularly limited as long as the exonuclease is derived from a filamentous fungus. Examples thereof include preferably an exonuclease derived from a fungus of the genus *Rhizopus,* more preferably one belonging to exonuclease 1 or exonuclease 2, further preferably an exonuclease derived from *Rhizopus oryzae* or *Rhizopus delemar.*

Preferably, an expression vector containing the polynucleotide encoding the exonuclease is transferred to host cells, and the exonuclease is coexpressed with the programmable DNA nuclease. Preferably, the polynucleotide encoding the exonuclease is operably linked to a promoter on the expression vector. The types of the expression vector and the promoter which can be used are the same as in the case of the DNA nuclease.

The single-stranded DNA used in the described methods is used as donor DNA for the genomic DNA of the host filamentous fungus. In the present specification, the "donor DNA" refers to a DNA fragment which is used for engineering genomic DNA of a host through homologous recombination. The donor DNA is single-stranded DNA containing two DNA sequences (also respectively referred to as an upstream homologous sequence and a downstream homologous sequence) homologous to the genomic DNA of a host. The upstream homologous sequence and the downstream homologous sequence contain regions homologous to an upstream region and a downstream region, respectively, of a site to be cleaved by the programmable DNA nuclease in the genomic DNA of the host. The cleavage site for the programmable DNA nuclease may be located between the upstream homologous sequence and the downstream homologous sequence. Alternatively, a portion or the whole of the cleavage site may be contained in any of the upstream homologous sequence and the downstream homologous sequence. The donor DNA may further have an insertion sequence to be inserted to the genomic DNA of the host filamentous fungus, between the upstream homologous sequence and the downstream homologous sequence. The insertion sequence is not particularly limited and may contain a sequence of an arbitrary gene region, for example, a coding region of the gene, a control region such as a promoter, an intron, and other noncoding regions and may further contain a selective marker gene such as a drug resistance gene. Preferably, in the insertion sequence, the coding region of the gene is operably linked to the control region.

The single-stranded DNA as donor DNA, which is transferred to a host, causes homologous recombination between the upstream region and the downstream region of the cleavage site in the genomic DNA of the host due to its upstream homologous sequence and downstream homologous sequence so that the sequence from the upstream region to the downstream region is replaced therewith. Thus, depending on the presence or absence of the insertion sequence in the single-stranded DNA, site-directed substitution, insertion or deletion of the sequence occurs in the genomic DNA of the host after the homologous recombination. More specifically, when the single-stranded DNA contains an insertion sequence, the insertion sequence is substituted for or inserted to the genomic DNA of the host. Alternatively, when the single-stranded DNA substantially has only the upstream homologous sequence and the downstream homologous sequence and contains no insertion sequence, deletion is brought about in the genomic DNA of the host.

The donor DNA can be constructed by preparing DNAs encoding the upstream homologous sequence and the downstream homologous sequence, and if necessary, the insertion sequence to be transferred to a host, and linking these DNAs. Those skilled in the art can obtain the DNA sequence of the surroundings of the cleavage site of the host genome, or the target region to be substituted by the single-stranded DNA, and design the DNAs of the upstream homologous sequence and the downstream homologous sequence on the basis thereof. The DNA encoding the insertion sequence may be DNA of one type of gene region or may be DNA containing two or more types of gene regions. For example, the insertion sequence may consist of a DNA fragment encoding the gene to be transferred to a host, and a DNA fragment encoding a marker gene such as a drug resistance gene. In a preferred example, a DNA fragment of the upstream homologous sequence, a DNA fragment encoding the insertion sequence, and a DNA fragment of the downstream homologous sequence are each constructed according to a standard method and then linked in order, to obtain the donor DNA.

The donor DNA may be first constructed as a double-stranded DNA and then denatured into single-stranded DNA. For example, a double-stranded DNA plasmid containing the upstream homologous sequence, the downstream homologous sequence, and the insertion sequence is constructed by cloning or the like and used as a template in PCR to obtain a double-stranded DNA fragment. The plasmid or the double-stranded DNA can be subjected to sonication, quenching following thermal denaturation, treatment with a strong alkali such as NaOH, etc. and thereby denatured into single-stranded DNA.

Alternatively, the donor DNA may be constructed as a single-stranded DNA from the start. The single-stranded DNA can be prepared by chemical synthesis (purchasable from Medical & Biological Laboratories Co., Ltd., etc.), asymmetric PCR, or the like. The single-stranded DNA can also be prepared by using a Ff phage such as M13 or a plasmid such as pBluescript having Fori, and *E. coli* such as JM109 having f factor. Further examples of the method for preparing the single-stranded DNA can include a method of biotinylating one of the primers, performing PCR, collecting the PCR product using streptavidin beads, then dissociating the PCR product into single-stranded DNAs, and eluting the unbiotinylated single-stranded DNA, and a method of phosphorylating one of the primers, and performing PCR, followed by treatment with Lambda Exonuclease.

Preferred examples of the method for preparing the single-stranded DNA include a method using a Ff phage and *E. coli* having f factor, a method using a biotinylated primer, and a method using a phosphorylated primer. Among them, a method using a phosphorylated primer is more preferred.

In the single-stranded DNA, each of the lengths of the upstream homologous sequence and the downstream homologous sequence is preferably 5 bp or longer, 10 bp or longer, 15 bp or longer, 20 bp or longer, 25 bp or longer, 30 bp or longer, 35 bp or longer, 40 bp or longer, 45 bp or longer, 50 bp or longer, 100 bp or longer, 250 bp or longer, 500 bp or longer, 750 bp or longer, or 1,000 bp or longer. When the single-stranded DNA has an insertion sequence between the upstream and downstream homologous sequences, the chain length of the insertion sequence can be 1 bp or longer and is preferably 0.3 kbp or longer, more preferably 1 kbp or longer, further preferably 3 kbp or longer and is preferably 50 kbp or shorter, more preferably 20 kbp or shorter, further preferably 10 kbp or shorter, still further preferably 9 kbp or shorter, still further, preferably 5 kbp or shorter. Alternatively, the chain length of the insertion sequence is preferably 1 bp or longer and 50 kbp or shorter, 0.3 kbp or longer and 50 kbp or shorter, 1 kbp or longer and 50 kbp or shorter, or 3 kbp or longer and 50 kbp or shorter, more preferably 1 bp or longer and 20 kbp or shorter, 0.3 kbp or longer and 20 kbp or shorter, 1 kbp or longer and 20 kbp or shorter, or 3 kbp or longer and 20 kbp or shorter, further preferably 1 bp or longer and 10 kbp or shorter, 0.3 kbp or longer and 10 kbp or shorter, 1 kbp or longer and 10 kbp or shorter, or 3 kbp or longer and 10 kbp or shorter, still further preferably 1 bp or longer and 9 kbp or shorter, 0.3 kbp or longer and 9 kbp or shorter, 1 kbp or longer and 9 kbp or shorter, or 3 kbp or longer and 9 kbp or shorter, still further preferably 1 bp or longer and 5 kbp or shorter, 0.3 kbp or longer and 5 kbp or shorter, 1 kbp or longer and 5 kbp or shorter, or 3 kbp or longer and 5 kbp or shorter.

Thus, the chain length of the single-stranded DNA used in the described methods is preferably 10 bp or longer, more preferably 20 bp or longer, further preferably 30 bp or longer, still further preferably 40 bp or longer, still further preferably 50 bp or longer, still further preferably 60 bp or longer, still further preferably 70 bp or longer, still further preferably 80 bp or longer, still further preferably 90 bp or longer, still further preferably 100 bp or longer, still further preferably 200 bp or longer, still further preferably 500 bp or longer, still further preferably 1,000 bp or longer, still further preferably 1,500 bp or longer, still further preferably 2,000 bp or longer, and is preferably 50 kbp or shorter, more preferably 20 kbp or shorter, further preferably 12 kbp or shorter, still further preferably 11 kbp or shorter.

Alternatively, the chain length of the single-stranded DNA used in the described methods is preferably 10 bp or longer and 50 kbp or shorter, 10 bp or longer and 20 kbp or shorter, 10 bp or longer and 12 kbp or shorter, 10 bp or longer and 11 kbp or shorter, 20 bp or longer and 50 kbp or shorter, 20 bp or longer and 20 kbp or shorter, 20 bp or longer and 12 kbp or shorter, 20 bp or longer and 11 kbp or shorter, 30 bp or longer and 50 kbp or shorter, 30 bp or longer and 20 kbp or shorter, 30 bp or longer and 12 kbp or shorter, 30 bp or longer and 11 kbp or shorter, 40 bp or longer and 50 kbp or shorter, 40 bp or longer and 20 kbp or shorter, 40 bp or longer and 12 kbp or shorter, 40 bp or longer and 11 kbp or shorter, 50 bp or longer and 50 kbp or shorter, 50 bp or longer and 20 kbp or shorter, 50 bp or longer and 12 kbp or shorter, 50 bp or longer and 11 kbp or shorter, 60 bp or longer and 50 kbp or shorter, 60 bp or longer and 20 kbp or shorter, 60 bp or longer and 12 kbp or shorter, 60 bp or longer and 11 kbp or shorter, 70 bp or longer and 50 kbp or shorter, 70 bp or longer and 20 kbp or shorter, 70 bp or longer and 12 kbp or shorter, 70 bp or longer and 11 kbp or shorter, 80 bp or longer and 50 kbp or shorter, 80 bp or longer and 20 kbp or shorter, 80 bp or longer and 12 kbp or shorter, 80 bp or longer and 11 kbp or shorter, 90 bp or longer and 50 kbp or shorter, 90 bp or longer and 20 kbp or shorter, 90 bp or longer and 12 kbp or shorter, 90 bp or longer and 11 kbp or shorter, 100 bp or longer and 50 kbp or shorter, 100 bp or longer and 20 kbp or shorter, 100 bp or longer and 12 kbp or shorter, 100 bp or longer and 11 kbp or shorter, 200 bp or longer and 50 kbp or shorter, 200 bp or longer and 20 kbp or shorter, 200 bp or longer and 12 kbp or shorter, 200 bp or longer and 11 kbp or shorter, 500 bp or longer and 50 kbp or shorter, 500 bp or longer and 20 kbp or shorter, 500 bp or longer and 12 kbp or shorter, 500 bp or longer and 11 kbp or shorter, 1,000 bp or longer and 50 kbp or shorter, 1,000 bp or longer and 20 kbp or shorter, 1,000 bp or longer and 12 kbp or shorter, 1,000 bp or longer and 11 kbp or shorter, 1,500 bp or longer and 50 kbp or shorter, 1,500 bp or longer and 20 kbp or shorter, 1,500 bp or longer and 12 kbp or shorter, 1,500 bp or longer and 11 kbp or shorter, 2,000 bp or longer and 50 kbp or shorter, 2,000 bp or longer and 20 kbp or shorter, 2,000 bp or longer and 12 kbp or shorter, or 2,000 bp or longer and 11 kbp or shorter.

The polynucleotide encoding the programmable DNA nuclease or the vector containing the polynucleotide, and the single-stranded DNA can be transferred to host cells by use of a general transforming method, for example, an electroporation method, a transformation method, a transfection method, a conjugation method, a protoplast method, a particle gun method, or an Agrobacterium method.

Recombinant cells which have undergone homologous recombination of interest by the transferred single-stranded DNA can be selected on the basis of deletion of the originally carried gene, expression of the transferred gene, expression of a marker gene, etc.

### [Examples]

The PCR primers used in the present Examples are shown in Tables 1 to 6.

**[Table 1]**

| Primer | Sequence (5'→3') | SEQ ID No. |
|---|---|---|
| oJK162 | cgagctcgaattatttaaatgaacagcaagttaataatctagaggg | 10 |
| oJK163 | tatgaccatgattacgatgagaggcaaaatgaagcgtac | 11 |
| oJK164 | atttaaataattcgagctcggtacccgggg | 12 |
| oJK165 | cgtaatcatggtcatagctg | 13 |
| oJK202 | tagagggaaaaagagagaattgaaatagg | 14 |
| oJK204 | ttttgttatttaattgtattaattgataatg | 15 |
| oJK205 | aattaaataacaaaatcattttaattacgcattttc | 16 |
| oJK216 | catgattacgcggccgcgccattataatgcactagtg | 17 |
| oJK210 | ctctttttccctctaatgagaggcaaaatgaagcgtac | 18 |
| oJK211 | atttaaatgtaatcatggtcatagctgtttc | 19 |
| trpC-lost-F | tttaaattagagggaaaaagagagaattgaaatag | 20 |
| trpC-lost-R | tccctctaatttaaatgaattcgagctcggtaccc | 21 |
| adhpro-R | ttttgttatttaattgtattaattgataatg | 22 |
| adhter-F | tcattttaattacgcattttcatttac | 23 |
| adhpro- TALEN-F | aattaaataacaaaaatggactacaaagaccatgacggtg | 24 |
| TAELN-adhter-R | gcgtaattaaaatgattaaaagtttatctcgccgttatta | 25 |
| adhpro-LifeTALEN-F | aattaaataacaaaaatgggaaaacctattcctaatcctctgctg | 26 |
| LifeTALEN-adhter-R | gcgtaattaaaatgatcagaagttgatctcgccgttgttgaactttc | 27 |
| pPTR1-sal1-F | gggtaccgagctcgaattc | 28 |
| pPTR1-sal1-R | ggggatcctctagagtcgac | 29 |
| saIl-ldhpro-F3 | ctctagaggatcccctaggtgtggctgtggtgaccatattg | 30 |
| Idhpro-R | gagaattatattgtaaagaaaaataaag | 31 |
| Idhpro-exo1-F2 | tacaatataattctcatgaaaatccaagttgcttctcctattgaccaatc | 32 |

**[Table 2]**

| Primer | Sequence (5'→3') | SEQ ID No. |
|---|---|---|
| exo1-pdcter-R2 | atgaattctaagattttatcttctttcatgagaaacactaaacttgataac | 33 |
| pdcTer-F | aatcttagaattcatctttttttg | 34 |
| pdcTer-sal1-R | tcgagctcggtacccactctaccgtctgctcttttgtct | 35 |
| adhpro-exo1-F | aattaaataacaaaaatgaaaatccaagttgcttctcctattgac | 36 |
| exo1-adhter-R | gcgtaattaaaatgattatcttctttcatgagaaacactaaacttg | 37 |
| pUC18-Pae1-F3 | ctgcaggtcgactctagaggatccccgggtaccg | 38 |
| pUC18-Hind3-R3 | gcttggcactggccgtcgttttacaacgtcgtgac | 39 |
| PDC1-upstr-F | cggccagtgccaagcgcagacttcaacagttggcttttttaagta | 40 |
| PDC1-upstr-R | cattttgcctctcatgtttttaaatttgttttgtagagtattgaata | 41 |
| trpCpro-R | gaacagcaagttaataatctagagggcgc | 42 |
| trpCter-F | atgagaggcaaaatgaagcgtacaaagag | 43 |
| PDC1-downstr-F | attaacttgctgttcaatcttagaattcattttttttttgtatcattcg | 44 |
| PDC1-downstr-R | agagtcgacctgcaggcgtcaataagagcttgaaggttggtgccggatc | 45 |
| PDC2-upstr-F | cggccagtgccaagcttatgggaaaaatgtgaaaatcatcccact | 46 |
| PDC2-upstr-R | cattttgcctctcatgtttagttcaaataatatttttttttgtga | 47 |
| PDC2-downstr-F | attaacttgctgttcagttctctttctgtaataatccttgatttc | 48 |
| PDC2-downstr-R | agagtcgacctgcagcctttttacagaaaacaagcacatctttac | 49 |
| trpC-inactive-F | taacgcatcagttccatcctgaaagtatcc | 50 |
| trpC-inactive-R | ggaactgatgcgttaaaaagaggggaaa | 51 |
| pdc1ter-F | aatcttagaattcattttttttttgtatc | 52 |
| trpC-adh1pro-F | attaacttgctgttctagagggaaaaagagagaattgaaatagg | 53 |
| FT1-pdc1Ter-R | atgaattctaagattttaatagaaaccctgcttaaatgcaagacc | 54 |
| PYC-pdc1Ter-R | atgaattctaagattttaggcttcctctttgacaaccttggccac | 55 |

**[Table 3]**

| Primer | Sequence (5'→3') | SEQ ID No. |
|---|---|---|
| PDC1-upstr-F2 | gcagacttcaacagttggcttttttaagta | 56 |
| PDC1-downstr-R2 | gcgtcaataagagcttgaaggttggtgccggatc | 57 |
| pdc1-up2 | cattcccacaggatttgtgc | 58 |
| trpC(d)-1 | gtgagatgttgatcatttgtacatg | 59 |
| pdc2-down1 | agaagacaacctagaccctc | 60 |
| trpC(d)-7 | atagctcttggtgggattcg | 61 |
| pdc1-down3 | gctgctcgagatcctccaaggtatc | 62 |
| trpC(d)-5 | ttcgaccaagggttccatac | 63 |
| PDC1-downstr-R-P | gcgtcaataagagcttgaaggttggtgccggatc | 64 |
| pdc1_750-F | tagattcaatttgattggataaagttcatc | 65 |
| pdc1_750-R-P | ataacaaacaatggctaaaagtggaccccc | 66 |
| pdc1_500-F | tttactagtttaaagcaaaaaacatgagca | 67 |
| pdc1_500-R-P | ttcaatttacatttctttatgaatatgcct | 68 |
| pdc1_250-F | gtgtttatctgttcacaagtactggtaagc | 69 |
| pdc1_250-R-P | ttcaatataaaccaagtccctaaaagaaat | 70 |
| pdc1_100-F | gctgatatgacattgcgacgaaaatagtat | 71 |
| pdc1_100-R-P | aaaaaaaaagcttattttcaaaaatatgat | 72 |
| pdc1_50-F | tcgttcaaaaaaaatcattattcaatactc | 73 |
| pdc1_50-R-P | gtaatgaagtatagaacgaatgatacaaaa | 74 |
| pdc1_45-F | caaaaaaaatcattattcaatactctacaa | 75 |
| pdc1_45-R-P | gaagtatagaacgaatgatacaaaaaaaaaat | 76 |

**[Table 4]**

| Primer | Sequence (5'→3') | SEQ ID No. |
|---|---|---|
| pdc1_40-F | aaaatcattattcaatactctacaaaacaa | 77 |
| pdc1_40-R-P | atagaacgaatgatacaaaaaaaaaatgaa | 78 |
| pdc1_35-F | cattattcaatactctacaaaacaaattta | 79 |
| pdc1_35-R-P | acgaatgatacaaaaaaaaaatgaattcta | 80 |
| pdc1_30-F | ttcaatactctacaaaacaaatttaaaaac | 81 |
| pdc1_30-R-P | tgatacaaaaaaaaaatgaattctaagatt | 82 |
| pdc1_25-F | tactctacaaaacaaatttaaaaacatgag | 83 |
| pdc1_25-R-P | caaaaaaaaaatgaattctaagattgaaca | 84 |
| pdc1_20-F | tacaaaacaaatttaaaaacatgagaggca | 85 |
| pdc1_20-R-P | aaaaaatgaattctaagattgaacagcaag | 86 |
| pdc1_15-F | aacaaatttaaaaacatgagaggcaaaatg | 87 |
| pdc1_15-R-P | atgaattctaagattgaacagcaagttaat | 88 |
| pdc1_10-F | atttaaaaacatgagaggcaaaatgaagcg | 89 |
| pdc1_10-R-P | ttctaagattgaacagcaagttaataatct | 90 |
| pdc1_5-F | aaaacatgagaggcaaaatgaagcgtacaa | 91 |
| pdc1_5-R-P | agattgaacagcaagttaataatctagagg | 92 |
| PDC2-upstr-F2 | ttatgggaaaaatgtgaaaatcatcccact | 93 |
| PDC2-downstr-R2-P | cctttttacagaaaacaagcacatctttac | 94 |
| trpC-ki-F2-P | cttttcatgacccaacaaatcagacac | 95 |

**[Table 5]**

| Primer | Sequence (5'→3') | SEQ ID No. |
|---|---|---|
| trpCter-R | atgagaggcaaaatgaagcgtacaaagag | 104 |
| trpCter-cicCpro-F | cattttgcctctcatcttacgcaggttgatagtagccgcc | 105 |
| cipCpro-adhter-R | gcgtaattaaaatgaggttagagtatgaagaaaaaaaaaa | 106 |
| trpC-lost-F2 | tttaaatcttacgcaggttgatagtagccgc | 107 |
| trpC-lost-R2 | tgcgtaagatttaaatgaattcgagctcggtac | 108 |
| cipCpro-R | ggttagagtatgaagaaaaaaaaaaaacg | 109 |
| cipCpro-LifeTALEN-F | cttcatactctaaccatgggaaaacctattcctaatcctctgctg | 110 |
| cipCpro-exo1-F | cttcatactctaaccatgaaaatccaagttgcttctccta | 111 |
| pdc3-upstr-F | cggccagtgccaagcccgtcaggggtgaatgagatatttt | 112 |
| pdc3-upstr-R2 | aaaagatgtgagttataaaaggatgatgcaagc | 113 |
| pdc3-downstr-F2 | taactcacatcttttattctttttctatccctc | 114 |
| pdc3-downstr-R | agagtcgacctgcagacctgttagaaaggtacatgcattc | 115 |
| pdc3-upstr-R | cattttgcctctcatgtgagttataaaaggatgatgcaag | 116 |
| pdc3-downstr-F | attaacttgctgttcatcttttattctttttctatccctc | 117 |
| pdc3-upstr-F2 | ccgtcaggggtgaatgagatatt | 118 |
| pdc3-downstr-R2-P | acctgttagaaaggtacatgcattc | 119 |
| pdc3-up | gacctcaatcactatccttgg | 120 |

**[Table 6]**

| Primer | Sequence (5'→3') | SEQ ID No. |
|---|---|---|
| pUC18-adh1pro-F | cggccagtgccaagcattattattagagggaaaaaaaagaaaga | 121 |
| adh1pro-pUC18-R | agagtcgacctgcagttttgttatttatttgtattaattgataa | 122 |
| adh1pro-adh1ter-F | aacaaaatcattttaattacgcattttcattttttactaa | 123 |
| adh1ter-pUC18-R | agagtcgacctgcagagcagaacatgagtctggaagcgagacac | 124 |
| adh1pro-adh1ter-R | taaaatgattttgttatttatttgtattaattgataatga | 125 |
| adh1pro(o)-R | ttttgttatttatttgtattaattgataatg | 126 |
| adh1ter(o)-F | tcattttaattacgcattttcattttttac | 127 |
| adh1 pro(o)-LifeTALEN-F | aaataaataacaaaaatgggaaaacctattcctaatcctctgct | 128 |
| LifeTALEN-adh1ter(o)-R | gcgtaattaaaatgatcagaagttgatctcgccgttgttgaact | 129 |
| IdhA-upstr-F | cggccagtgccaagcaaaagaataagaaaagatgtgtcag | 130 |
| IdhA-upstr-R2 | taaattaaggacttgagcttgaacgatgtcag | 131 |
| IdhA-downstr-F2 | caagtccttaatttacaaataataaatcatgtt | 132 |
| IdhA-downstr-R | agagtcgacctgcagaacgacaaacatggctatcaaggga | 133 |
| IdhA-upstr-R | tgtgcaagtgaacaaaggacttgagcttgaacgatgtcag | 134 |
| IdhA-downstr-F | gctcttgacaatgcataatttacaaataataaatcatgtt | 135 |
| ade1pro-R | tgcattgtcaagagcgtgtcgcaac | 136 |
| ade1ter-F | ttgttcacttgcacagcgtgatatgcaag | 137 |
| IdhA-upstr-F2 | aaaagaataagaaaagatgtgtcaggac | 138 |
| IdhA-downstr-R2-P | aacgacaaacatggctatcaagggaac | 139 |
| IdhA-up | gaaactacagtattccctcgtg | 140 |
| ade1-15 | tgcttccatatgtcaataggc | 141 |
| exo1-adhter-R2 | gcgtaattaaaatgattatcttctttcatgagaaacacta | 142 |

### Example 1 Preparation of mutant of genus Rhizopus

### (1) Preparation of tryptophan auxotrophic strain

A *Rhizopus delemar* JCM (Japan Collection of Microorganisms/Riken, Japan) 5557 strain (hereinafter, referred to as a 5557 strain) or a tryptophan auxotrophic strain derived from the 5557 strain was used as a parent strain of homologous recombinants. The tryptophan auxotrophic strain was obtained by screening from among strains mutated by ion beam irradiation of the 5557 strain. The ion beam irradiation was performed at the facility of Takasaki ion accelerators for advanced radiation application (TIARA), Takasaki Advanced Radiation Research Institute, National Institutes for Quantum and Radiological Science and Technology. The strain was irradiated with 100 to 1,250 G ray at an energy of 220 MeV with ¹²C⁵⁺ accelerated using an AVF cyclotron. Spores were collected from the irradiated fungal cells. From among them, a *Rhizopus delemar* 02T6 strain which had a one-base deletion mutation in the trpC gene region and exhibited tryptophan auxotrophy was obtained. The 5557 strain or the 02T6 strain was used as a parent strain for preparation of homologous recombinants of the fungus of the genus *Rhizopus* in subsequent Examples.

### (2) Plasmid vector preparation

A DNA fragment of the trpC gene region was synthesized by PCR using the genomic DNA of the 5557 strain as a template with primers of oJK162 (SEQ ID NO: 10) and oJK163 (SEQ ID NO: 11). Next, a DNA fragment was amplified by PCR using a plasmid pUC18 as a template with primers of oJK164 (SEQ ID NO: 12) and oJK165 (SEQ ID NO: 13). These two fragments were ligated using In-Fusion HD Cloning Kit (Clontech Laboratories, Inc.) to prepare a plasmid pUC18-trpC.

Subsequently, a promoter fragment and a terminator fragment of adh1 were amplified by PCR using the genomic DNA of the 5557 strain as a template with primers of oJK202 (SEQ ID NO: 14) and oJK204 (SEQ ID NO: 15) and primers of oJK205 (SEQ ID NO: 16) and oJK216 (SEQ ID NO: 17), respectively. Next, a DNA fragment was amplified by PCR using the plasmid pUC18-trpC constructed as described above as a template with primers of oJK210 (SEQ ID NO: 18) and oJK211 (SEQ ID NO: 19). These three fragments were ligated using In-Fusion HD Cloning Kit (Clontech Laboratories, Inc.) to prepare a plasmid pUC18-trpC-Padh-Tadh. In the obtained plasmid, the adh1 promoter and terminator were placed in order downstream of the trpC gene region.

Further, a plasmid vector in which the trpC gene region was removed from pUC18-trpC-Padh-Tadh was prepared. Specifically, a DNA fragment was amplified by PCR using the pUC18-trpC-Padh-Tadh constructed as described above as a template with primers of trpC-lost-F (SEQ ID NO: 20) and trpC-lost-R (SEQ ID NO: 21). This fragment was ligated using In-Fusion HD Cloning Kit (Clontech Laboratories, Inc.) to prepare a plasmid pUC18-Padh-Tadh.

### (3) Preparation of TALEN for pdc1, pdc2 and trpC gene disruption

TALENs targeting the pdc1, pdc2 or trpC gene locus were prepared. The TALEN preparation was requested to Transposagen Biopharmaceuticals, Inc. for the TALENs targeting the pdc1 gene locus, and Life Technologies Corp. for the TALENs targeting the pdc2 or trpC gene locus to obtain Custom XTN TALEN (trade name of TALEN provided by Transposagen Biopharmaceuticals, Inc.) or GeneArt Precision TALs (trade name of TALEN provided by Life Technologies Corp.). These kits each contain two polynucleotides encoding Left-TALEN and Right-TALEN for the target gene. The kit targeting the pdc1 gene (SEQ ID NO: 1) contains LeftTALEN-pdc1 (SEQ ID NO: 2) and RightTALEN-pdc1 (SEQ ID NO: 3) which encode TALENs targeting the sequence of 5'-TGCCTGCTATTAAAATCG-3' (SEQ ID NO: 96) in the sense strand of the pdc1 gene and the sequence of 5'-TTGATTTCCTTAAGACGG-3' (SEQ ID NO: 97) in the antisense strand thereof, respectively. The kit targeting the pdc2 gene (SEQ ID NO: 4) contains LeftTALEN-pdc2 (SEQ ID NO: 5) and RightTALEN-pdc2 (SEQ ID NO: 6) which encode TALENs targeting the sequence of 5'-TGGTGTTGCTGGTTCTTAT-3' (SEQ ID NO: 98) in the sense strand of the pdc2 gene and the sequence of 5'-TGCCGACAATGTGAATCAC-3' (SEQ ID NO: 99) in the antisense strand thereof, respectively. The kit targeting the trpC gene (SEQ ID NO: 7) contains LeftTALEN-trpC (SEQ ID NO: 8) and RightTALEN-trpC (SEQ ID NO: 9) which encode TALENs targeting the sequence of 5'-TGCCAAGGCGCCAATGTAG-3' (SEQ ID NO: 100) in the sense strand of the trpC gene and the sequence of 5'-TCGGAAATGGTGATTTTGT-3' (SEQ ID NO: 101) in the antisense strand thereof, respectively.

The polynucleotide encoding LeftTALEN for the pdc1 gene was inserted to the expression vector pUC18-trpC-Padh-Tadh for *R. delemar* prepared in the paragraph (2) to prepare a vector for expression of TALEN under control of the adh1 promoter and the adh1 terminator. Specifically, a vector fragment was amplified by PCR using pUC18-trpC-Padh-Tadh as a template with primers of adhpro-R (SEQ ID NO: 22) and adhter-F (SEQ ID NO: 23). Subsequently, a LeftTALEN-pdc1 fragment was amplified by PCR using LeftTALEN-pdc1 as a template with primers of adhpro-TALEN-F (SEQ ID NO: 24) and TALEN-adhter-R (SEQ ID NO: 25). These two fragments contained regions overlapping with each other by 15 bases. These two fragments were ligated using In-Fusion HD cloning kit (Clontech Laboratories, Inc.) to obtain a plasmid padh-LeftTALEN-pdc1 containing LeftTALEN-pdc1.

Likewise, a vector for expression of TALEN under control of the adh1 promoter and the adh1 terminator was prepared from the polynucleotide encoding Left-TALEN for the pdc1 gene using the expression vector pUC18-Padh-Tadh for *R. delemar* prepared in the paragraph (2). Specifically, a vector fragment was amplified by PCR using pUC18-Padh-Tadh as a template with primers of adhpro-R (SEQ ID NO: 22) and adhter-F (SEQ ID NO: 23). Subsequently, a LeftTALEN-pdc1 fragment was amplified by PCR using LeftTALEN-pdc1 as a template with primers of adhpro-TALEN-F (SEQ ID NO: 24) and TALEN-adhter-R (SEQ ID NO: 25), and ligated with the vector fragment to obtain a plasmid padh-LeftTALEN-pdc1-2 containing LeftTALEN-pdc1.

A vector for expression of TALEN under control of the adh1 promoter and the adh1 terminator was prepared from the polynucleotide encoding Right-TALEN for the pdc1 gene using the expression vector pUC18-Padh-Tadh for R. *delemar* prepared in the paragraph (2). Specifically, a vector fragment was amplified by PCR using pUC18-Padh-Tadh as a template with primers of adhpro-R (SEQ ID NO: 22) and adhter-F (SEQ ID NO: 23). Subsequently, a RightTALEN-pdc1 fragment was amplified by PCR using RightTALEN-pdc1 as a template with primers of adhpro-TALEN-F (SEQ ID NO: 24) and TALEN-adhter-R (SEQ ID NO: 25), and ligated with the vector fragment to obtain a plasmid padh-RightTALEN-pdc1 containing RightTALEN-pdc1.

Vectors for expression of TALEN under control of the adh1 promoter and the adh1 terminator was prepared from the polynucleotides encoding Left-TALEN and Right-TALEN for the pdc2 gene using the expression vector pUC18-Padh-Tadh for *R. delemar* prepared in the paragraph (2). Specifically, a vector fragment was amplified by PCR using pUC18-Padh-Tadh as a template with primers of adhpro-R (SEQ ID NO: 22) and adhter-F (SEQ ID NO: 23). Subsequently, a LeftTALEN-pdc2 or RightTALEN-pdc2 fragment was amplified by PCR using LeftTALEN-pdc2 or RightTALEN-pdc2 as a template with primers of adhpro-LifeTALEN-F (SEQ ID NO: 26) and LifeTALEN-adhter-R (SEQ ID NO: 27), and ligated with the vector fragment to obtain a plasmid padh-LeftTALEN-pdc2 or padh-RightTALEN-pdc2 containing LeftTALEN-pdc2 or RightTALEN-pdc2.

Likewise, vectors for expression of TALEN under control of the adh1 promoter and the adh1 terminator was prepared from the polynucleotides encoding Left-TALEN and Right-TALEN for the trpC gene using the expression vector pUC18-Padh-Tadh for *R. delemar* prepared in the paragraph (2). Specifically, a vector fragment was amplified by PCR using pUC18-Padh-Tadh as a template with primers of adhpro-R (SEQ ID NO: 22) and adhter-F (SEQ ID NO: 23). Subsequently, a LeftTALEN-trpC or RightTALEN-trpC fragment was amplified by PCR using LeftTALEN-trpC or RightTALEN-trpC as a template with primers of adhpro-LifeTALEN-F (SEQ ID NO: 26) and LifeTALEN-adhter-R (SEQ ID NO: 27), and ligated with the vector fragment to obtain a plasmid padh-LeftTALEN-trpC or padh-RightTALEN-trpC containing LeftTALEN-trpC or RightTALEN-trpC.

### (4) Preparation of exonuclease expression vector

A pUC18 vector fragment was amplified by PCR using a plasmid pUC18 (Takara Bio Inc.) as a template with primers of pPTR1-sal1-F (SEQ ID NO: 28) and pPTR1-sal1-R (SEQ ID NO: 29). Also, a ldh promoter fragment was amplified using a purified genome solution of a *Rhizopus oryzae* NRBC5384 strain (hereinafter, referred to as a 5384 strain) as a template with primers of sal1-ldhpro-F3 (SEQ ID NO: 30) and ldhpro-R (SEQ ID NO: 31). An exonuclease gene fragment was amplified using the same template as above with primers of ldhpro-exo1-F2 (SEQ ID NO: 32) and exo1-pdcter-R2 (SEQ ID NO: 33). A pdc terminator fragment was amplified using the same template as above with primers of pdcTer-F (SEQ ID NO: 34) and pdcTer-sal1-R (SEQ ID NO: 35). These four amplified fragments were ligated using In-Fusion HD cloning kit (Clontech Laboratories, Inc.) to prepare a plasmid pldh-exo1.

Also, an exonuclease gene fragment was amplified using a purified genome solution of the 5384 strain as a template with primers of adhpro-exo1-F (SEQ ID NO: 36) and exo1-adhter-R (SEQ ID NO: 37). A vector fragment was amplified by PCR using pUC18-trpC-Padh-Tadh as a template with primers of adhpro-R (SEQ ID NO: 22) and adhter-F (SEQ ID NO: 23). These two amplified fragments were ligated using In-Fusion HD cloning kit (Clontech Laboratories, Inc.) to prepare a plasmid padh-exo1.

### (5) Preparation of plasmid for trpC knock-in targeting each gene locus

A plasmid ptrpC-knock-in (pdc1) for removing pdc1 gene ORF and knocking-in the trpC gene region at the pdc1 gene locus was prepared. Specifically, a pUC18 vector fragment amplified using pUC18 as a template with primers of pUC18-Pae1-F3 (SEQ ID NO: 38) and pUC18-Hind3-R3 (SEQ ID NO: 39), a promoter site fragment of the pdc1 gene amplified using the genome of the 5557 strain as a template with primers of PDC1-upstr-F (SEQ ID NO: 40) and PDC1-upstr-R (SEQ ID NO: 41), a trpC gene region fragment amplified using the genome of the 5557 strain as a template with primers of trpCpro-R (SEQ ID NO: 42) and trpCter-F (SEQ ID NO: 43), a terminator site fragment of the pdc1 gene amplified using the genome of the 5557 strain as a template with primers of PDC1-downstr-F (SEQ ID NO: 44) and PDC1-downstr-R (SEQ ID NO: 45) were ligated using In-Fusion HD Cloning Kit (Clontech Laboratories, Inc.) to construct a ptrpC-knock-in (pdc1).

Likewise, a plasmid ptrpC-knock-in (pdc2) for removingpdc2 gene ORF and knocking-in the trpC gene region at the pdc2 gene locus was prepared. Specifically, a pUC18 vector fragment amplified using pUC18 as a template with primers of pUC18-Pae1-F3 (SEQ ID NO: 38) and pUC18-Hind3-R3 (SEQ ID NO: 39), a promoter site fragment of the pdc2 gene amplified using the genome of the 5557 strain as a template with primers of PDC2-upstr-F (SEQ ID NO: 46) and PDC2-upstr-R (SEQ ID NO: 47), a trpC gene region fragment amplified using the genome of the 5557 strain as a template with primers of trpCpro-R (SEQ ID NO: 42) and trpCter-F (SEQ ID NO: 43), a terminator site fragment of the pdc2 gene was amplified using the genome of the 5557 strain as a template with primers of PDC2-downstr-F (SEQ ID NO: 48) and PDC2-downstr-R (SEQ ID NO: 49 were ligated using In-Fusion HD Cloning Kit (Clontech Laboratories, Inc.) to construct ptrpC-knock-in (pdc2).

Subsequently, a plasmid ptrpC-knock-in (trpC) for disrupting the trpC gene at the trpC gene locus by knocking-in a trpC gene region (SEQ ID NO: 7) having a 500 bp deletion from the start codon in the trpC gene region was prepared. Specifically, a fragment was amplified using pUC18-trpC-Padh-Tadh as a template with primers of trpC-inactive-F (SEQ ID NO: 50) and trpC-inactive-R (SEQ ID NO: 51) and ligated at both ends using In-Fusion HD Cloning Kit (Clontech Laboratories, Inc.) to construct ptrpC-knock-in (trpC) containing a trpC gene sequence having a 500 bp deletion from the start codon.

### (6) Preparation of plasmid for (trpC + FT1) knock-in targeting pdc1 gene locus

A plasmid p(trpC + FT1)-knock-in (pdc1) was prepared by transferring the adh1 promoter and FT1 gene on the 5' end of the trpC gene region sequence in the plasmid ptrpC-knock-in (pdcl). Specifically, a vector fragment amplified using ptrpC-knock-in (pdc1) as a template with primers of trpCpro-R (SEQ ID NO: 42) and pdc1ter-F (SEQ ID NO: 52), a fragment containing the adh1 promoter and the FT1 gene amplified using a FT1 plasmid (plasmid harboring the FT1 gene (SEQ ID NO: 102) downstream of the adh1 promoter of pUC18-trpC-Padh-Tadh) as a template with primers of trpC-adh1pro-F (SEQ ID NO: 53) and FT1-pdc1Ter-R (SEQ ID NO: 54) were ligated using In-Fusion HD Cloning Kit (Clontech Laboratories, Inc.) to construct p(trpC + FT1)-knock-in (pdc1).

### (7) Preparation of plasmid for (trpC + PYC) knock-in targeting pdc1 gene locus

p(trpC + PYC)-knock-in (pdc1) was prepared in the same way as in the paragraph (6). Specifically, a vector fragment amplified using ptrpC-knock-in (pdc1) as a template with primers of trpCpro-R (SEQ ID NO: 42) and pdc1ter-F (SEQ ID NO: 52), a fragment containing the adh1 promoter and the PYC gene amplified using a PYC plasmid (plasmid harboring the PYC gene (SEQ ID NO: 103) downstream of the adh1 promoter of pUC18-trpC-Padh-Tadh) as a template with primers of trpC-adh1pro-F (SEQ ID NO: 53) and PYC-pdc1Ter-R (SEQ ID NO: 55) were ligated using In-Fusion HD Cloning Kit (Clontech Laboratories, Inc.) to construct p(trpC + PYC)-knock-in (pdc1).

### (8) Preparation of double-stranded DNA

Double-stranded DNA was prepared according to No. 1 of Table 7. Specifically, a DNA fragment was amplified using the plasmid ptrpC-knock-in (pdc1) as a template with primers of PDC1-upstr-F2 (SEQ ID NO: 56) and PDC1-downstr-R2 (SEQ ID NO: 57). The template was degraded by Dpn1 (Toyobo Co., Ltd.) treatment. Subsequently, the product was purified by phenol/chloroform/isoamyl alcohol treatment and ethanol precipitation treatment. The purified product was dissolved in an appropriate amount of DNase free water to obtain a double-stranded DNA solution for pdc1 gene disruption.

### (9) Preparation of single-stranded DNA

Single-stranded DNAs were prepared according to No. 2 to 20 of Table 7. Specifically, a DNA fragment having a length of "Insert length" of Table 7 was amplified using "Template" of each condition of Table 7 as a template with "Primer 1" and "Primer 2". The template was degraded by Dpn1 (Toyobo Co., Ltd.) treatment. Subsequently, the product was purified by phenol/chloroform/isoamyl alcohol treatment and ethanol precipitation treatment. The purified product was further treated using Lambda Exonuclease (NEW ENGLAND BioLabs Inc.) and then purified in the same way as above to obtain single-stranded DNA. The Lambda Exonuclease treatment was performed overnight at 37°C.

**[Table 7]**

| No. | DNA | Primer 1 | Primer 2 | Template | Gene locus | Insert length (bp) | Homologous sequence length (bp) |
|---|---|---|---|---|---|---|---|
| 1 | **Double-stranded** | PDC1-upstr-F2 | PDC1-downstr-R2 | ptrpC-knock-in(pdc1) | pdc1 | 4298 | 1000 |
| 2 | **Single-stranded** | PDC1-upstr-F2 | PDC1-downstr-R-P | ptrpC-knock-in (pdc1) | pdc1 | 4298 | 1000 |
| 3 | **Single-stranded** | PDC1-upstr-F2 | PDC1-downstr-R-P | p(trpC+FT1)-knock-in(pdc1) | pdc1 | 5811 | 1000 |
| 4 | **Single-stranded** | PDC1-upstr-F2 | PDC1-downstr-R-P | p(trpC+PYC)-knock-in(pdc1) | pdc1 | 8838 | 1000 |
| 5 | **Single-stranded** | pdc1_750-F | pdc1_750-R-P | ptrpC-knock-in (pdc1) | pdc1 | 4298 | 750 |
| 6 | **Single-stranded** | pdc1_500-F | pdc1_500-R-P | ptrpC-knock-in (pdcl) | pdc1 | 4298 | 500 |
| 7 | **Single-stranded** | pdc1-250-F | pdc1_250-R-P | ptrpC-knock-in(pdc1) | pdc1 | 4298 | 250 |
| 8 | **Single-stranded** | pdc1_100-F | pdc1_100-R-P | ptrpC-knock-in (pdc1) | pdc1 | 4298 | 100 |
| 9 | **Single-stranded** | pdc1_50-F | pdc1_50-R-P | ptrpC-knock-in (pdc1) | pdc1 | 4298 | 50 |
| 10 | **Single-stranded** | pdc1_45-F | pdc1_45-R-P | ptrpC-knock-in (pdc1) | pdc1 | 4298 | 45 |
| 11 | **Single-stranded** | pdc1_40-F | pdc1_40-R-P | ptrpC-knock-in (pdc1) | pdc1 | 4298 | 40 |
| 12 | **Single-stranded** | pdc1_35-F | pdc1_35-R-P | ptrpC-knock-in (pdc1) | pdc1 | 4298 | 35 |
| 13 | **Single-stranded** | pdc1-30-F | pdc1_30-R-P | ptrpC-knock-in (pdc1) | pdc1 | 4298 | 30 |
| 14 | **Single-stranded** | pdc1_25-F | pdc1_25-R-P | ptrpC-knock-in (pdc1) | pdc1 | 4298 | 25 |
| 15 | **Single-stranded** | pdc1-20-F | pdc1_20-R-P | ptrpC-knock-in (pdc1) | pdc1 | 4298 | 20 |
| 16 | **Single-stranded** | pdc1-15-F | pdc1_15-R-P | ptrpC-knock-in (pdc1) | pdc1 | 4298 | 15 |
| 17 | **Single-stranded** | pdc1-10-F | pdc1_10-R-P | ptrpC-knock-in (pdc1) | pdc1 | 4298 | 10 |
| 18 | **Single-stranded** | pdc1_5-F | pdc1_5-R-P | pt rpC-knock-in (pdc1) | pdc1 | 4298 | 5 |
| 19 | **Single-stranded** | PDC2-upstr-F2 | PDC2-downstr-R2-P | ptrpC-knock-in (pdc2) | pdc2 | 4298 | 1000 |
| 20 | **Single-stranded** | trpC-ki-F2-P | trpCpro-R | ptrpC-knock-in (trpC) | trpC | 3798 | 1000 |

| | | | | | | | |
|---|---|---|---|---|---|---|---|
| *Primer 2 of No. 2 to 19 was 5'-terminally phosphorylated. Primer 1 of No. 20 was 5'-terminally phosphorylated. "Homologous sequence length" represents that homologous sequences having the length shown in the table were present on both the upstream and downstr eam ends of the single-stranded DNA or the double-stranded DNA. | | | | | | | |

### (10) Gene transfer using particle gun

Each single-stranded DNA or the double-stranded DNA prepared in the paragraph (8) or (9) (No. 1 to 20 of Table 7), each Left-TALEN expression vector and each Right-TALEN expression vector prepared in the paragraph (3), and each exonuclease expression vector prepared in the paragraph (4) were mixed to prepare a DNA solution.

The TALEN expression vectors used were padh-LeftTALEN-pdc1 and padh-RightTALEN-pdc1 for No. 1 and 2, padh-LeftTALEN-pdc1-2 and padh-RightTALEN-pdc1 for No. 3 to 18, padh-LeftTALEN-pdc2 and padh-RightTALEN-pdc2 for No. 19, and padh-LeftTALEN-trpC and padh-RightTALEN-trpC for No. 20. The exonuclease expression vector used was pldh-exo1 for No. 1 and 2, and padh-exo1 for others. The gene transfer plasmids were subjected to restriction enzyme treatment only for the conditions of No. 1 and 2. Specifically, padh-LeftTALEN-pdc1, padh-LeftTALEN-pdc1-2 and padh-RightTALEN-pdc1 were treated with a restriction enzyme ScaI, and the plasmid pldh-exo1 was treated with a restriction enzyme Pst1. The concentration ratio among the Left-TALEN expression vector, the Right-TALEN expression vector, the exonuclease expression vector, and the single-stranded DNA or the double-stranded DNA in the DNA solution was set to approximately 2:4:2:1 for No. 1 and 2 and approximately 1:1:1:2 for No. 3 to 20.

10 µL of each prepared DNA solution (approximately 1 to 3 µg/µL) was added to and mixed with 100 µL of a gold particle solution (60 mg/mL, INBIO GOLD, particle size: 1 µm). Further, 40 µL of 0.1 M spermidine was added thereto, and the mixture was well stirred by vortex. 100 µL of 2.5 M CaCl₂ was added thereto, and the mixture was stirred for 1 minute by vortex and then centrifuged at 6,000 rpm for 30 seconds to remove a supernatant. To the obtained precipitates, 200 µL of 70% EtOH was added, and the mixture was stirred for 30 seconds by vortex and then centrifuged at 6,000 rpm for 30 seconds to remove a supernatant. The obtained precipitates were resuspended in 100 µL of 100% EtOH.

The spores of the 02T6 strain or the 5557 strain obtained in the paragraph (1) were subjected to gene transfer using each DNA-gold particle solution described above and GDS-80 (particle gun from Nepa Gene Co., Ltd.). The 02T6 strain was used for the conditions of No. 1 to 19, and the spores after the gene transfer were statically cultured at 30°C for approximately 1 week on an inorganic agar medium (20 g/L glucose, 1 g/L ammonium sulfate, 0.6 g/L potassium dihydrogen phosphate, 0.25 g/L magnesium sulfate heptahydrate, 0.09 g/L zinc sulfate heptahydrate, and 15 g/L agar). The spores of the 5557 strain was used for the condition of No. 20, and the spores after the gene transfer were statically cultured at 30°C for approximately 1 week on PDB medium (39 g/L PDB) .

### (11) Selection of pdc1 gene-deficient strain (No. 1 to No. 18)

The spores were collected from the fungal cells cultured in the paragraph (10). Fungal strains were isolated using an inorganic agar medium (20 g/L glucose, 1 g/L ammonium sulfate, 0.6 g/L potassium dihydrogen phosphate, 0.25 g/L magnesium sulfate heptahydrate, 0.09 g/L zinc sulfate heptahydrate, and 15 g/L agar) adjusted to pH 3. A portion of mycelia of the grown fungal strains was scraped off using a toothpick, then suspended in 10 mM Tris-HCl (pH 8.5), and incubated at 95°C for 10 minutes. Then, the suspension was appropriately diluted with 10 mM Tris-HCl (pH 8.5) to prepare a genome template solution for colony PCR. Colony PCR was performed using the genome template solution, primers pdc1-up2 (SEQ ID NO: 58) and trpC(d)-1 (SEQ ID NO: 59), and KOD FX Neo (Toyobo Co., Ltd.). The colony PCR using these primers amplifies a DNA fragment having an appropriate length if the trpC gene fragment or another gene fragment with the trpC gene is knocked-in at the pdc1 gene locus. By the colony PCR, a fungal strain with the DNA amplification fragment obtained was obtained as a knock-in strain (pdc1 gene-deficient strain).

### (12) Selection of pdc2 gene-deficient strain (No. 19)

The spores were collected from the fungal cells cultured in the paragraph (10). Fungal strains were isolated using an inorganic agar medium (20 g/L glucose, 1 g/L ammonium sulfate, 0.6 g/L potassium dihydrogen phosphate, 0.25 g/L magnesium sulfate heptahydrate, 0.09 g/L zinc sulfate heptahydrate, and 15 g/L agar) adjusted to pH 3. A portion of mycelia of the grown fungal strains was scraped off using a toothpick, then suspended in 10 mM Tris-HCl (pH 8.5), and incubated at 95°C for 10 minutes. Then, the suspension was appropriately diluted with 10 mM Tris-HCl (pH 8.5) to prepare a genome template solution for colony PCR. Colony PCR was performed using the genome template solution, primers pdc2-down1 (SEQ ID NO: 60) and trpC(d)-7 (SEQ ID NO: 61), and KOD FX Neo (Toyobo Co., Ltd.). The colony PCR using these primers amplifies a DNA fragment having an appropriate length if the trpC gene fragment is knocked-in at the pdc2 gene locus. By the colony PCR, a fungal strain with the DNA amplification fragment obtained was obtained as a knock-in strain (pdc2 gene-deficient strain).

### (13) Selection of trpC gene-deficient strain (No. 20)

The spores were collected from the fungal cells cultured in the paragraph (10), and cultured in 5-FAA medium (10 g/L glucose, 3.36 g/L Yeast Nitrogen Base w/o amino acids, 0.03 g/L tryptophan, 5 g/L 5-fluoroanthranilic acid, and 15 g/L agar) adjusted to pH 6. The grown fungal strains were subcultured in 5-FAA medium. A portion of mycelia of the grown fungal strains was scraped off using a toothpick, then suspended in 10 mM Tris-HCl (pH 8.5), and incubated at 95°C for 10 minutes. Then, the suspension was appropriately diluted with 10 mM Tris-HCl (pH 8.5) to prepare a genome template solution for colony PCR. Colony PCR was performed using the genome template solution, primers pdc1-down3 (SEQ ID NO: 62) and trpC(d)-5 (SEQ ID NO: 63), and KOD FX Neo (Toyobo Co., Ltd.). The band position of a DNA amplification fragment is shifted by the colony PCR using these primers if the trpC gene fragment having a 500 bp deletion is knocked-in at the trpC gene locus. By the colony PCR, a fungal strain with the band of the DNA amplification fragment shifted was obtained as a knock-in strain (trpC gene-deficient strain).

### (14) Comparison of homologous recombination efficiency

### (a) Single-stranded DNA vs. double-stranded DNA

Homologous recombination efficiency was compared between the double-stranded DNA and the single-stranded DNA (No. 1 and 2 of Table 7) used as donor DNA. The results are shown in Table 8. A homologously recombinant strain having a long-chain sequence transferred was unable to be obtained by using the double-stranded DNA, whereas the homologously recombinant strain having a long-chain sequence transferred was successfully obtained by using the single-stranded DNA. Furthermore, a homologously recombinant strain having a long-chain sequence transferred of approximately 8.8 kbp at the longest was successfully obtained by using the single-stranded DNA as donor DNA (No. 2 to 4 of Table 7).

**[Table 8]**

| Experimental condition No. | The number of Isolated strains | The number of homologously recombinant strains | Homologous recombination efficiency (%) |
|---|---|---|---|
| 1 | 108 | 0 | 0 |
| 2 | 8 | 1 | 12.5 |

### (b) Length of region homologous to host genomic DNA

Influence of the chain lengths of regions homologous to the host genomic DNA in the single-stranded DNA on homologous recombination efficiency was studied (No. 2, 8, and 9 of Table 7). The results are shown in Table 9.

**[Table 9]**

| Experimental condition No. | Homologous sequence length (bp) | The number of Isolated strains | The number of homologously recombinant strains | Homologous recombination efficiency (%) |
|---|---|---|---|---|
| 2 | 1000 | 8 | 1 | 12.5 |
| 8 | 100 | 24 | 22 | 92 |
| 9 | 50 | 24 | 19 | 79 |

### (15) Obtainment of pdc3 gene-deficient strain

### (a) Preparation of plasmid vector

A plasmid vector was prepared by changing the adh1 promoter of the pUC18-trpC-Padh-Tadh constructed in the paragraph (2) to cipC promoter. Specifically, a vector fragment was amplified by PCR using the pUC18-trpC-Padh-Tadh as a template with primers of adhter-F (SEQ ID NO: 23) and trpCter-R (SEQ ID NO: 104). Also, a cipC promoter region fragment was amplified by PCR using the genomic DNA of the 5557 strain as a template with of primers trpCter-cicCpro-F (SEQ ID NO: 105) and cipCpro-adhter-R (SEQ ID NO: 106). These two fragments were ligated using In-Fusion HD Cloning Kit (Clontech Laboratories, Inc.) to prepare a plasmid pUC18-trpC-PcipC-Tadh.

Subsequently, a plasmid vector in which the trpC gene region was removed from the pUC18-trpC-PcipC-Tadh constructed as described above was prepared. Specifically, a DNA fragment was amplified by PCR using the pUC18-trpC-PcipC-Tadh as a template with primers of trpC-lost-F2 (SEQ ID NO: 107) and trpC-lost-R2 (SEQ ID NO: 108). This fragment was ligated using In-Fusion HD Cloning Kit (Clontech Laboratories, Inc.) to prepare a plasmid pUC18-PcipC-Tadh.

### (b) Preparation of TALEN for pdc3 gene disruption

TALENs targeting the pdc3 gene locus were prepared. The TALEN preparation was requested to Life Technologies Corp. to obtain GeneArt PerfectMatch TALs (trade name of TALEN provided by Life Technologies Corp.). This kit contains two polynucleotides encoding Left-TALEN and Right-TALEN for the target gene. The kit targeting the pdc3 gene (SEQ ID NO: 143) contains LeftTALEN-pdc3 (SEQ ID NO: 144) and RightTALEN-pdc3 (SEQ ID NO: 145) which encode TALENs targeting the sequence of 5'-CCGGAATCGACACGATTTT-3' (SEQ ID NO: 146) in the sense strand of the pdc3 gene and the sequence of 5'-CGTAACTTACCATATTGTA-3' (SEQ ID NO: 147) in the antisense strand thereof, respectively.

The polynucleotide encoding Left-TALEN for the pdc3 gene was inserted to the expression vector pUC18-PcipC-Tadh for *R. delemar* prepared in the paragraph (15)(a) to prepare a vector for expression of TALEN under control of the cipC promoter and the adh1 terminator. Specifically, a vector fragment was amplified by PCR using pUC18-PcipC-Tadh as a template with primers of cipCpro-R (SEQ ID NO: 109) and adhter-F (SEQ ID NO: 23). Subsequently, a LeftTALEN-pdc3 fragment was amplified by PCR using LeftTALEN-pdc3 as a template with primers of cipCpro-LifeTALEN-F (SEQ ID NO: 110) and LifeTALEN-adhter-R (SEQ ID NO: 27). These two fragments contained regions overlapping with each other by 15 bases. These two fragments were ligated using In-Fusion HD cloning kit (Clontech Laboratories, Inc.) to obtain a plasmid pcipC-LeftTALEN-pdc3 containing LeftTALEN-pdc3.

Likewise, the polynucleotide encoding RightTALEN for the pdc3 gene was inserted to the expression vector pUC18-PcipC-Tadh for *R. delemar* prepared in the paragraph (15) (a) to prepare a vector for expression of TALEN under control of the cipC promoter and the adh1 terminator. Specifically, a vector fragment was amplified by PCR using pUC18-PcipC-Tadh as a template with primers of cipCpro-R (SEQ ID NO: 109) and adhter-F (SEQ ID NO: 23). Subsequently, a RightTALEN-pdc3 fragment was amplified by PCR using RightTALEN-pdc3 as a template with primers of cipCpro-LifeTALEN-F (SEQ ID NO: 110) and LifeTALEN-adhter-R (SEQ ID NO: 27). These two fragments contained regions overlapping with each other by 15 bases. These two fragments were ligated using In-Fusion HD cloning kit (Clontech Laboratories, Inc.) to obtain a plasmid pcipC-RightTALEN-pdc3 containing RightTALEN-pdc3.

### (c) Preparation of exonuclease expression vector

An exonuclease gene fragment was amplified by PCR using the plasmid pldh-exo1 prepared in the paragraph (4) as a template with primers of cipCpro-exo1-F (SEQ ID NO: 111) and exo1-adhter-R2 (SEQ ID NO: 142). A vector fragment was amplified by PCR using the plasmid pUC18-PcipC-Tadh prepared in the paragraph (15)(a) as a template with primers of cipCpro-R (SEQ ID NO: 109) and adhter-F (SEQ ID NO: 23). These two fragments contained regions overlapping with each other by 15 bases. These two fragments were ligated using In-Fusion HD cloning kit (Clontech Laboratories, Inc.) to obtain a plasmid pcipC-exo1.

### (d) Preparation of plasmid for trpC knock-in targeting pdc3 gene locus

A plasmid ptrpC-knock-in (pdc3) for removing pdc3 gene ORF and knocking-in the trpC gene region at the pdc3 gene locus was prepared. Specifically, a pUC18 vector fragment was amplified using pUC18 as a template with primers of pUC18-Pae1-F3 (SEQ ID NO: 38) and pUC18-Hind3-R3 (SEQ ID NO: 39). A pdc3 gene promoter fragment was amplified using the genomic DNA of the 5557 strain as a template with primers of pdc3-upstr-F (SEQ ID NO: 112) and pdc3-upstr-R2 (SEQ ID NO: 113). A pdc3 gene terminator fragment was amplified using the genomic DNA of the 5557 strain as a template with primers of pdc3-downstr-F2 (SEQ ID NO: 114) and pdc3-downstr-R (SEQ ID NO: 115). These three fragments were ligated using In-Fusion HD Cloning Kit (Clontech Laboratories, Inc.) to prepare pknock-in (pdc3).

Subsequently, a DNA fragment was amplified using pknock-in (pdc3) as a template with primers of pdc3-upstr-R (SEQ ID NO: 116) and pdc3-downstr-F (SEQ ID NO: 117). A trpC gene region fragment was amplified using the genomic DNA of the 5557 strain as a template with primers of trpCpro-R (SEQ ID NO: 42) and trpCter-F (SEQ ID NO: 43). These two fragments were ligated using In-Fusion HD Cloning Kit (Clontech Laboratories, Inc.) to prepare a ptrpC-knock-in (pdc3).

### (e) Preparation of single-stranded DNA

Single-stranded DNA was obtained by the same procedures as in the paragraph (9) except that: plasmid ptrpC-knock-in (pdc3) was used as a PCR template; and pdc3-upstr-F2 (SEQ ID NO: 118) and pdc3-downstr-R2-P (SEQ ID NO: 119; 5'-terminally phosphorylated) were used as PCR primers.

### (f) Gene transfer using particle gun

By the same procedures as in the paragraph (10), a DNA-gold particle solution containing single-stranded DNA was prepared, and gene transfer to the spores of the 02T6 strain was performed using this solution, followed by culturing of the obtained spores. The single-stranded DNA used was the single-stranded DNA prepared in the paragraph (15) (e). The TALEN expression vectors used were the pcipC-LeftTALEN-pdc3 and the pcipC-RightTALEN-pdc3 prepared in the paragraph (15) (b). The exonuclease expression vector used was the pcipC-exo1 prepared in the paragraph (15)(c). The concentration ratio among pcipC-LeftTALEN-pdc3, pcipC-RightTALEN-pdc3, pcipC-exo1, and single-stranded DNA in the DNA solution was set to approximately 1:1:1:2.

### (g) Selection of pdc3 gene-deficient strain

By the same procedures as in the paragraph (11), the spores were collected from the fungal cells cultured in the paragraph (15)(f), and the isolation of fungal strains and the preparation of a genome template solution were performed. Subsequently, a pdc3 gene-deficient strain with the trpC gene region fragment knocked-in at the pdc3 gene locus was selected by colony PCR using the genome template solution as a template. The colony PCR was performed using the genome template solution, primers pdc3-up (SEQ ID NO: 120) and trpC(d)-1 (SEQ ID NO: 59), and KOD FX Neo (Toyobo Co., Ltd.). The colony PCR using these primers amplifies a DNA fragment having an appropriate length if the trpC gene region fragment is knocked-in at the pdc3 gene locus. By the colony PCR, a fungal strain with the DNA amplification fragment obtained was obtained as a knock-in strain (pdc3 gene-deficient strain).

### (h) Homologous recombination efficiency at pdc3 gene locus

The obtained pdc3 gene-deficient strain was examined for homologous recombination efficiency (Table 10).

**[Table 10]**

| Upstream homologous sequence length (bp) | Downstream homologous sequence length (bp) | The number of isolated strains | The number of homologously recombinant strains | Homologous recombination efficiency (%) |
|---|---|---|---|---|
| 1000 | 950 | 20 | 8 | 40 |

### Example 2 Preparation of mutant of genus Rhizopus (Rhizopus oryzae)

### (1) Preparation of adenine auxotrophic strain

A *Rhizopus oryzae* NRBC5384 strain (hereinafter, referred to as a 5384 strain) was used as a parent strain of homologous recombinants. An adenine auxotrophic strain was obtained by screening from among strains mutated by the UV irradiation of spores of the 5384 strain. The UV irradiation was performed by irradiation for 1 minute using a bactericidal lamp GL15 (Hitachi Appliances, Inc.). The irradiated spores were collected. From among them, a *Rhizopus oryzae* AM002 strain which had a mutation in the ade1 gene region and exhibited adenine auxotrophy was obtained. The *Rhizopus oryzae* AM002 strain was used as a parent strain for preparation of homologous recombinants of *Rhizopus oryzae* in subsequent Examples.

### (2) Preparation of plasmid vector for Rhizopus oryzae

A promoter fragment of adh1 was amplified by PCR using the genomic DNA of the 5384 strain as a template with primers of pUC18-adh1pro-F (SEQ ID NO: 121) and adh1pro-pUC18-R (SEQ ID NO: 122). Next, a DNA fragment was amplified by PCR using pUC18 as a template with primers of pUC18-Pae1-F3 (SEQ ID NO: 38) and pUC18-Hind3-R3 (SEQ ID NO: 39). These two fragments were ligated using In-Fusion HD Cloning Kit (Clontech Laboratories, Inc.) to prepare plasmid pUC18-Padh (RO).

Subsequently, a terminator fragment of adh1 was amplified by PCR using the genomic DNA of the 5384 strain as a template with primers of adh1pro-adh1ter-F (SEQ ID NO: 123) and adh1ter-pUC18-R (SEQ ID NO: 124). Further, a DNA fragment was amplified by PCR using pUC18-Padh (RO) as a template with primers of pUC18-Pae1-F3 (SEQ ID NO: 38) and adh1pro-adh1ter-R (SEQ ID NO: 125). These two fragments were ligated using In-Fusion HD Cloning Kit (Clontech Laboratories, Inc.) to prepare a plasmid pUC18-Padh-Tadh (RO).

### (3) Preparation of TALEN for ldhA gene disruption.

TALENs targeting the ldhA gene locus were prepared. The TALEN preparation was requested to Life Technologies Corp. to obtain GeneArt Precision TALs (trade name of TALEN provided by Life Technologies Corp.). This kit contains two polynucleotides encoding Left-TALEN and Right-TALEN for the target gene. The kit targeting the ldhA gene (SEQ ID NO: 148) contains LeftTALEN-ldhA (SEQ ID NO: 149) and RightTALEN-ldhA (SEQ ID NO: 150) which encode TALENs targeting the sequence of 5'-TGCAGATGCTGCCAGTATA-3' (SEQ ID NO: 151) in the sense strand of the ldhA gene and the sequence of 5'-TCCTCTGCGCTACCTGCTC-3' (SEQ ID NO: 152) in the antisense strand thereof, respectively.

The polynucleotide encoding Left-TALEN for the ldhA gene was inserted to the expression vector pUC18-Padh-Tadh (RO) for *R. oryzae* prepared in the paragraph (2) to prepare a vector for expression of TALEN under control of the adh1 promoter and the adh1 terminator. Specifically, a vector fragment was amplified by PCR using pUC18-Padh-Tadh (RO) as a template with primers of adh1pro(o)-R (SEQ ID NO: 126) and adh1ter(o)-F (SEQ ID NO: 127). Subsequently, a LeftTALEN-ldhA fragment was amplified by PCR using LeftTALEN-ldhA as a template with primers of adh1pro(o)-LifeTALEN-F (SEQ ID NO: 128) and LifeTALEN-adhlter(o)-R (SEQ ID NO: 129). These two fragments contained regions overlapping with each other by 15 bases. These two fragments were ligated using In-Fusion HD cloning kit (Clontech Laboratories, Inc.) to obtain a plasmid padh-LeftTALEN-ldhA (RO) containing LeftTALEN-ldhA.

Likewise, the polynucleotide encoding Right-TALEN for the ldhA gene was inserted to the expression vector pUC18-Padh-Tadh (RO) to prepare a vector for expression of TALEN under control of the adh1 promoter and the adh1 terminator. Specifically, a vector fragment was amplified by PCR using pUC18-Padh-Tadh (RO) as a template with primers of adh1pro(o)-R (SEQ ID NO: 126) and adhlter(o)-F (SEQ ID NO: 127). Subsequently, a RightTALEN-ldhA fragment was amplified by PCR using RightTALEN-ldhA as a template with primers of adh1pro(o)-LifeTALEN-F (SEQ ID NO: 128) and LifeTALEN-adh1ter(o)-R (SEQ ID NO: 129). These two fragments contained regions overlapping with each other by 15 bases. These two fragments were ligated using In-Fusion HD cloning kit (Clontech Laboratories, Inc.) to obtain a plasmid padh-RightTALEN-ldhA (RO) containing RightTALEN-ldhA.

### (4) Preparation of plasmid for ade1 knock-in targeting ldhA gene locus

A plasmid pade1-knock-in (ldhA) for partially removing ldhA gene ORF and knocking-in the ade1 gene region at the ldhA gene locus was prepared. Specifically, a pUC18 vector fragment was amplified using pUC18 as a template with primers of pUC18-Pae1-F3 (SEQ ID NO: 38) and pUC18-Hind3-R3 (SEQ ID NO: 39). A ldhA gene promoter site fragment containing a portion of the ldhA gene was amplified using the genomic DNA of the 5384 strain as a template with primers of ldhA-upstr-F (SEQ ID NO: 130) and ldhA-upstr-R2 (SEQ ID NO: 131). A ldhA gene terminator site fragment was amplified using the genomic DNA of the 5384 strain as a template with primers of ldhA-downstr-F2 (SEQ ID NO: 132) and ldhA-downstr-R (SEQ ID NO: 133). These three fragments were ligated using In-Fusion HD Cloning Kit (Clontech Laboratories, Inc.) to prepare pknock-in (ldhA).

Subsequently, a DNA fragment was amplified using pknock-in (ldhA) as a template with primers of ldhA-upstr-R (SEQ ID NO: 134) and ldhA-downstr-F (SEQ ID NO: 135). An ade1 gene region fragment was amplified using the genomic DNA of the 5384 strain as a template with primers of ade1pro-R (SEQ ID NO: 136) and ade1ter-F (SEQ ID NO: 137). These two fragments were ligated using In-Fusion HD Cloning Kit (Clontech Laboratories, Inc.) to prepare pade1 knock-in (ldhA).

### (5) Preparation of double-stranded DNA

A DNA fragment was amplified using the plasmid pade1 knock-in (ldhA) as a template with primers of ldhA-upstr-F2 (SEQ ID NO: 138) and ldhA-downstr-R2-P (SEQ ID NO: 139; 5'-terminally phosphorylated). The template was degraded by Dpn1 (Toyobo Co., Ltd.) treatment. Subsequently, the product was purified by phenol/chloroform/isoamyl alcohol treatment and ethanol precipitation treatment. The purified product was dissolved in an appropriate amount of DNase free water to obtain a double-stranded DNA solution for ldhA gene disruption.

### (6) Preparation of single-stranded DNA

Subsequently, the double-stranded DNA obtained in the paragraph (5) was treated using Lambda Exonuclease (NEW ENGLAND BioLabs Inc.) and then purified in the same way as above to obtain single-stranded DNA. The Lambda Exonuclease treatment was performed overnight at 37°C.

### (7) Gene transfer using particle gun

By the same procedures as in Example 1(10), a DNA-gold particle solution containing the single-stranded or double-stranded DNA was prepared, and gene transfer to the spores of the AM002 strain was performed using this solution, followed by culturing of the obtained spores. The single-stranded DNA or the double-stranded DNA used was the single-stranded DNA or the double-stranded DNA prepared in the paragraph (6) or (5). The TALEN expression vectors used were the padh-LeftTALEN-ldhA (RO) and the padh-RightTALEN-ldhA (RO) prepared in the paragraph (3). The exonuclease expression vector used was the pldh-exo1 prepared in Example 1(4). The concentration ratio among padh-LeftTALEN-ldhA (RO), padh-RightTALEN-ldhA (RO), pldh-exo1, and single-stranded DNA or double-stranded DNA in the DNA solution was set to approximately 1:1:1:2.

### (8) Selection of ldhA gene-deficient strain

The spores were collected from the fungal cells cultured in the paragraph (7). The isolation of fungal strains and the preparation of a genome template solution were performed by the same procedures as in Example 1(11). Subsequently, a ldhA gene-deficient strain with the ade1 gene region fragment knocked-in at the ldhA gene locus was selected by colony PCR using the genome template solution as a template. The colony PCR was performed using the genome template solution, primers ldhA-up (SEQ ID NO: 140) and ade1-15 (SEQ ID NO: 141), and KOD FX Neo (Toyobo Co., Ltd.). The colony PCR using these primers amplifies a DNA fragment having an appropriate length if the ade1 gene region fragment is knocked-in at the ldhA gene locus. By the colony PCR, a fungal strain with the DNA amplification fragment obtained was obtained as a knock-in strain (ldhA gene-deficient strain).

### (9) Comparison of homologous recombination efficiency in Rhizopus oryzae

### (a) Single-stranded DNA vs. double-stranded DNA

Homologous recombination efficiency was compared between the double-stranded DNA and the single-stranded DNA used as donor DNA. The results are shown in Table 11. A homologously recombinant strain having a long-chain sequence transferred was unable to be obtained by using the double-stranded DNA, whereas the homologously recombinant strain having a long-chain sequence transferred was successfully obtained by using the single-stranded DNA.

**[Table 11]**

| Experimental condition | Homologous sequence length (bp) | The number of Isolated strains | The number of homologously recombinant strains | Homologous recombination efficiency (%) |
|---|---|---|---|---|
| Double-stranded DNA | 1000 | 24 | 0 | 0 |
| Single-stranded DNA | 1000 | 24 | 18 | 75 |

| | | | | |
|---|---|---|---|---|
| *"Homologous sequence length" represents that homologous sequences having the length shown in the table were present on both the upstream and downstream ends of the single-stranded DNA or the double-stranded DNA. | | | | |

## Claims

1. A method for producing a mutant filamentous fungus, comprising transferring a programmable DNA nuclease and single-stranded DNA into a host filamentous fungus, and substituting an upstream region and a downstream region of a cleavage site for the programmable DNA nuclease in genomic DNA of the host by the single-stranded DNA through homologous recombination, wherein the filamentous fungus is a fungus of the genus *Rhizopus.*

2. The method according to claim 1, wherein the length of the single-stranded DNA is 10 bp or longer and 50 kbp or shorter.

3. The method according to claim 1 or 2, wherein the single-stranded DNA comprises two DNA sequences respectively comprising regions homologous to the upstream region and the downstream region of the cleavage site in the genomic DNA of the host.

4. The method according to claim 3, wherein each of the lengths of the two DNA sequences is 5 bp or longer.

5. The method according to any one of claims 1 to 4, wherein the single-stranded DNA further comprises a sequence to be inserted to the genomic DNA of the host.

6. The method according to claim 5, wherein the length of the sequence to be inserted is 1 bp or longer and 50 kbp or shorter.

7. The method according to any one of claims 1 to 6, wherein the programmable DNA nuclease is an artificial DNA nuclease based on TALEN, CRISPR-Cas9, CRISPR-Cpfl or ZFN technology.

8. The method according to any one of claims 1 to 7, wherein the fungus of the genus *Rhizopus* is *Rhizopus delemar* or *Rhizopus oryzae.*

## Patentansprüche

1. Verfahren zur Herstellung eines mutanten filamentösen Pilzes, umfassend das Transferieren einer programmierbaren DNA-Nuclease und einzelsträngiger DNA in einen filamentösen Pilz-Wirt und das Substituieren einer Region stromaufwärts und einer Region stromabwärts einer Schnittstelle für die programmierbare DNA-Nuclease in der genomischen DNA des Wirts mit der einzelsträngigen DNA durch homologe Rekombination, wobei der filamentöse Pilz ein Pilz der Gattung *Rhizopus* ist.

2. Verfahren nach Anspruch 1, wobei die Länge der einzelsträngigen DNA 10 bp oder länger oder 50 kbp oder kürzer ist.

3. Verfahren nach Anspruch 1 oder 2, wobei die einzelsträngige DNA jeweils zwei DNA-Sequenzen umfasst, die Regionen umfassen, die mit der Region stromaufwärts und der Region stromabwärts der Schnittstelle in der genomischen DNA des Wirts homolog sind.

4. Verfahren nach Anspruch 3, wobei jede der Längen der zwei DNA-Sequenzen 5 bp oder länger ist.

5. Verfahren nach einem der Ansprüche 1 bis 4, wobei die einzelsträngige DNA des Weiteren eine Sequenz umfasst, die in die genomische DNA des Wirts zu inserieren ist.

6. Verfahren nach Anspruch 5, wobei die Länge der zu inserierenden Sequenz 1 bp oder länger und 50 kbp oder kürzer ist.

7. Verfahren nach einem der Ansprüche 1 bis 6, wobei die programmierbare DNA-Nuclease eine künstliche DNA-Nuclease ist, die auf TALEN, CRISPR-Cas9, CRlSPR-CpfI oder ZFN-Technologie basiert.

8. Verfahren nach einem der Ansprüche 1 bis 7, wobei der Pilz der Gattung *Rhizopus Rhizopus delemar* oder *Rhizopus oryzae* ist.

## Revendications

1. Méthode pour produire un champignon filamenteux mutant, comprenant le transfert d'une ADN nucléase programmable et d'un ADN simple brin dans un champignon filamenteux hôte, et le remplacement d'une région amont et d'une région aval d'un site de coupure par l'ADN nucléase programmable dans l'ADN génomique de l'hôte au moyen de l'ADN simple brin par l'intermédiaire d'une recombinaison homologue, dans laquelle le champignon filamenteux est un champignon du genre *Rhizopus.*

2. Méthode selon la revendication 1, dans laquelle la longueur de l'ADN simple brin est de 10 pb ou plus et de 50 kpb ou moins.

3. Méthode selon la revendication 1 ou 2, dans laquelle l'ADN simple brin comprend deux séquences d'ADN comprenant respectivement des régions homologues de la région amont et de la région aval du site de coupure dans l'ADN génomique de l'hôte.

4. Méthode selon la revendication 3, dans laquelle chacune des longueurs des deux séquences d'ADN est de 5 pb ou plus.

5. Méthode selon l'une quelconque des revendications 1 à 4, dans laquelle l'ADN simple brin comprend en outre une séquence devant être insérée dans l'ADN génomique de l'hôte.

6. Méthode selon la revendication 5, dans laquelle la longueur de la séquence devant être insérée est de 1 pb ou plus et de 50 kpb ou moins.

7. Méthode selon l'une quelconque des revendications 1 à 6, dans laquelle l'ADN nucléase programmable est une ADN nucléase artificielle basée sur la technologie TALEN, CRISPR-Cas9, CRISPR-Cpfl ou ZFN.

8. Méthode selon l'une quelconque des revendications 1 à 7, dans laquelle le champignon du genre *Rhizopus* est *Rhizopus delemar* ou *Rhizopus oryzae.*
